Europäisches Patentamt

European Patent Office

Office européen des brevets

(11) Veröffentlichungsnummer: **0 458 058 B1**

## (12) EUROPÄISCHE PATENTSCHRIFT

(45) Veröffentlichungstag der Patentschrift: **30.08.95**

(51) Int. Cl.6: **G01N 27/14**, G01N 33/00

(21) Anmeldenummer: **91106006.9**

(22) Anmeldetag: **16.04.91**

(54) **Verfahren für den Betrieb einer Messanordnung zum Nachweis des Anteils von brennbaren Gasen.**

(30) Priorität: **10.05.90 DE 4014930**

(43) Veröffentlichungstag der Anmeldung:
**27.11.91 Patentblatt 91/48**

(45) Bekanntmachung des Hinweises auf die
Patenterteilung:
**30.08.95 Patentblatt 95/35**

(84) Benannte Vertragsstaaten:
**DE FR GB**

(56) Entgegenhaltungen:
**EP-A- 0 080 406**
**FR-A- 2 537 722**
**GB-A- 2 022 269**
**GB-A- 2 185 577**
**US-A- 4 151 738**

(73) Patentinhaber: **Drägerwerk Aktiengesellschaft**

**D-23542 Lübeck (DE)**

(72) Erfinder: **Kauschke, Wolgang, Dr.**
**Lindenstrasse 17**
**W-2400 Lübeck (DE)**
Erfinder: **Thoren, Werner, Dr.rer.nat.**
**Gertrudenstrasse 1a**
**W-2400 Lübeck (DE)**

EP 0 458 058 B1

**Beschreibung**

Die Erfindung betrifft ein Verfahren für den Betrieb einer Meßanordnung zum Nachweis des Anteils von brennbaren Gasen in einem Gasgemisch mit einer Anzeigeeiheit, einer Meßeinrichtung, einem Detektorelement und einem ansteuerbaren Kompensatorelement.

Zur Messung der Konzentration von brennbaren Gasen werden Gassensoren eingesetzt, die einen Katalysator enthalten, der auf eine bestimmte Temperatur (z.B. 500 °C) erhitzt wird, wodurch die brennbaren Gase unter Verbrauch eines Teiles des im Meßgas befindlichen Sauerstoffs an der Sensoroberfläche katalytisch verbrennen.

Als Meßeinrichtung wird dazu üblicherweise ein aktiver Sensor (Detektor) und ein passiver Sensor (Kompensator) in einer oder zwei getrennten Meßbrücken angeordnet. Der Detektor reagiert mit dem zu messenden Gas, während der Kompensator im wesentlichen nur zum Ausgleich von Umgebungseinflüssen dient. Er wird auf die gleiche Temperatur wie der Detektor aufgeheizt. Die der Konzentration des brennbaren Gases proportionale Meßspannung unterhalb der unteren Explosionsgrenze (UEG) ist gleich der Differenz zwischen Detektor- und Kompensatorspannung.

Ein Meßverfahren zum Messen der Menge der in einem Gas vorhandenen Bestandteile mit einer dazugehörigen Meßschaltung ist aus der EP-B1 18221 bekanntgeworden.

Bei dem bekannten Meßverfahren sind das Detektorelement und das Kompensatorelement in separaten Meßbrücken angeordnet und an eine gemeinsame Spannungsversorgung angeschlossen. Die Speiseströme werden in der Weise gesteuert, daß sowohl das Detektor- als auch das Kompensatorelement bei vorgewählten Temperaturen gehalten werden. Hierzu ist jede Meßbrücke mit einer Schalteinrichtung als Zweipunktregler in der Spannungsversorgung versehen, die den Speisestrom eine Zeitlang unterbricht, wenn die vorgewählte Temperatur überschritten wurde. Die Kalibration von Detektor- und Kompensatorelement erfolgt in der Weise, daß die Ausgangsspannungen bei einem Bezugsgas, das frei von brennbaren Bestandteilen ist, auf gleiche Größe abgeglichen werden. Für bestimmte Anwendungsfälle ist auch vorgesehen, daß Detektor- und Kompensatorelement mit Spannungen ungleicher Größe und damit bei ungleichen Temperaturen arbeiten, wobei die unterschiedlichen Ausgangsspannungen mit einer Spannungsstelleinrichtung am Summationspunkt der beiden Meßbrücken ausgeglichen werden, so daß die nachgeschalteten Verstärker eine abgeglichene Meßbrücke sehen. Dieser Abgleich geschieht wieder mit einem Bezugsgas, das frei von brennbaren Bestandteilen ist.

Bei dem bekannten Meßverfahren ist es von Nachteil, daß das Detektor- und Kompensatorelement nur bei festen, vorbestimmten Temperaturen betrieben werden können und die Leistungsaufnahme - speziell in der Bereitschaftsphase ohne Anwesenheit brennbarer Gase - genauso groß ist wie in der Meßphase mit brennbaren Gasen. Besonders bei tragbaren Geräten müssen zur Deckung des Strombedarfs dann unnötig große Batterien mitgeführt werden, um den Leistungsbedarf des Meßgerätes zu decken. Dadurch werden diese Geräte unhandlich und schwer.

Aus der FR-A-2537722 ist ein Verfahren zur Bestimmung der Explosionsgrenze eines Gasgemisches bekanntgeworden. Hier werden ein Detektorelement und ein Kompensationselement jeweils an eine Spannungsversorgung angeschlossen und die beiden Speiseströme nach dem Einschaltzeitpunkt auf konstante Temperatur eingeregelt. Die Differenz der Spannungsverläufe am Detektorelement und am Kompensationselement in Abhängigkeit von der Zeit wird von einer Meßeinrichtung registriert. Aus dem Spannungsverlauf wird über Stützwerte eine Extrapolationsfunktion errechnet, die zur Bestimmung der Explosionsgrenze des Gasgemisches dient.

Bei der bekannten Anordnung ist es von Nachteil, daß das Detektorelement und auch das Kompensatorelement während der Meßphase immer an den Betriebsstrom angeschlossen sind und somit ständig die volle Leistungsaufnahme benötigt wird. Außerdem ist das System während der Meßpausen - in denen das Detektorelement nicht an den Speisestrom angeschlossen ist - nicht meßbereit.

EP-A-80406 offenbart einen konventionellen Wärmetönungssensor für explosive Gase mit einer Wheatstonebrücke, deren einer Arm von einem katalytischen Detektorelement, an dem das zu messende Gas oxydiert, in Serie mit einem Kompensationselement gebildet wird. Bei einer ersten Meßphase wird die Meßbrücke von einer ersten Spannung gespeist und das Vorzeichen des Brückensignals ausgewertet. Ist das Signal positiv, ist der Gehalt an explosivem Gas erhöht. Ist das Signal negativ, wird die Brücke in einer zweiten Meßphase mit einer zweiten höheren Spannung gespeist und das Vorzeichen und/oder die Höhe dieses zweiten Brückensignals ausgewertet.

Der vorliegenden Erfindung liegt die Aufgabe zugrunde, ein kontinuierlich arbeitendes Meßverfahren zum Nachweis von brennbaren Gasen so zu verbessern, daß die Leistungsaufnahme minimiert und an die zu messende Konzentration des brennbaren Gases adaptiert wird.

Die Lösung der Aufgabe erfolgt durch ein Verfahren, welches während einer ersten Betriebsphase das Detektorelement mit dem Detektor-Betriebsstrom ID speist, während das Kompensatorelement mit einem Kompensator-Teilbetriebsstrom IK1 betrieben wird und aus den Spannungen am Detektorelement UD und Kompensatorelement UK ein erstes Meßsignal U1 zur Gasanalyse gebildet wird und daß in einer zweiten Betriebsphase das Kompensatorelement vom Einschaltzeitpunkt t0 bis zum Zeitpunkt t1 in einer Teil-Aufheizphase an den Kompensator-Betriebsstrom IK angeschlossen ist und der stationäre Endwert der Kompensatorspannung UK(ts) am Ende der Aufheizphase aus dem Anstieg der Kompensatorspannung UK(t) im Zeitintervall t0 bis t1 mit einer Verarbeitungsfunktion extrapoliert wird und mit der extrapolierten Endspannung UK(ts) und der Spannung am Detektorelement UD ein zweites Meßsignal U2 zur Gasanalyse gebildet wird.

Der Vorteil des Verfahrens besteht im wesentlichen darin, daß das Kompensatorelement, das zum Ausgleich von Umwelteinflüssen dient, während der ersten Betriebsphase - der Bereitschaftsphase - nur mit dem Kompensator-Teilbetriebsstrom IK1 gespeist wird. Das Detektorelement, das immer an den Detektor-Betriebsstrom ID angeschlossen ist, ist ständig meßbereit und detektiert die Anwesenheit eines brennbaren Gases im Meßgas. Da das Kompensatorelement in der ersten Betriebsphase mit dem Kompensator-Teilbetriebsstrom IK1 versorgt wird, ist eine Konzentrationsmessung nur mit eingeschränkter Meßgenauigkeit möglich. Das aus dem Spannungsabfall am Detektorelement UD und Kompensatorelement UK gebildete Meßsignal U1 - das der Quotient der beiden Spannungen sein kann - wird in der nachgeschalteten Meßeinrichtung ausgewertet und die ermittelte Konzentration zur Anzeige gebracht. Die Konzentration ist ein Maß für die Brennbarkeit des Gasgemisches. Wird durch das Meßsignal U1 ein in der Meßeinrichtung gespeichertes Umschaltkriterium ausgelöst, schaltet die Steuereinheit in der zweiten Betriebsphase - der sog. Meßphase - für ein vorbestimmtes Zeitintervall t0 bis t1 - mit t0 als Einschaltzeitpunkt - den Kompensator-Betriebsstrom IK an das Kompensatorelement. Aus dem Spannungsverlauf am Kompensatorelement im Zeitintervall von t0 bis t1, wird dann mit einer Verarbeitungsfunktion auf den Endwert der Spannung am Kompensatorelement extrapoliert, der sich sonst erst nach einer Einschaltdauer ts - von z.B. einer halben Stunde - einstellt. Die Länge der Einschaltdauer ts bis zum Erreichen des stationären Endwertes ist im wesentlichen abhängig von konstruktiven Merkmalen des Sensorkopfes und dem verwendeten Kompensatortyp. Durch die Extrapolation der Spannung auf den Endwert kann der Aufheizvorgang des Kompensatorelementes vorzeitig abgebrochen werden, und es ist eine Konzentrationsmessung mit einer gegenüber der ersten Betriebsphase erhöhter Meßgenauigkeit möglich. Trotz des Umschaltens von der Bereitschaftsin die Meßphase ist die Meßanordnung ständig meßbereit. In der zweiten Betriebsphase wird das zweite Meßsignal U2 aus der extrapolierten Spannung UK(ts) am Kompensatorelement und der Spannung am Detektorelement UD gebildet.

Für die Erfassung der Temperatur T innerhalb des Sensorkopfes in der Umgebung von Detektorelement und Kompensatorelement ist ein Temperaturfühler vorgesehen. Zweckmäßige Bauformen sind ein Platinwiderstand (Pt 100, Pt200) oder ein Halbleiterwiderstand (KTY), da sie im relevanten Temperaturbereich eine ausreichende Steilheit der Kennlinie aufweisen. Das Temperatursignal wird der Meßeinrichtung zugeführt und bei der Berechnung der Verarbeitungsfunktion verwendet. Es dient dazu, die Temperaturabhängigkeit der Verarbeitungsfunktion auszugleichen.

Das Umschaltkriterium zwischen der ersten und zweiten Betriebsphase ist zweckmäßigerweise so ausgeführt, daß dieses nach Überschreiten eines ersten Grenzwertes DU1 des ersten Meßsignals U1 ausgelöst wird. Der erste Grenzwert DU1 liegt im Bereich von 1% bis 20% - vorzugsweise 5% - der unteren Explosionsgrenze (UEG). Dieser Bereich gibt die Empfindlichkeit des Umschaltkriteriums an. Für die Mehrzahl der bekanntenbrennbaren Gase ist 5%(UEG) ein signifikanter Schwellwert oberhalb der statistischen Schwankungen durch Umgebungseinflüsse. Ist die zweite Betriebsphase durch Überschreiten des ersten Grenzwertes DU1 ausgelöst worden und die Gaskonzentration im Sensorkopf während der zweiten Betriebsphase unverändert geblieben, muß durch die Meßeinrichtung der weitere Ablauf so beeinflußt werden, daß die zweite Betriebsphase nicht anschließend wieder neu ausgelöst wird. Dieser Ablauf kann beispielsweise so erfolgen, daß der erste Grenzwert DU1 für eine befristete Zeit angehoben wird, oder erst nach einem von der Meßeinrichtung vorgebbaren Zeitintervall wieder ausgelöst werden kann.

Als Alternative - oder in Kombination - ist es Zweckmäßig, die zweite Betriebsphase nach einer von der Meßeinrichtung vorgebbaren Zeit t2 auszulösen und den Meßwert mit dem aus der ersten Betriebsphase vorliegenden Wert zu vergleichen und auf Plausibilität zu prüfen. Liegt die Abweichung außerhalb der in der Meßeinrichtung gespeicherten zulässigen Fehlerbandbreite, wird das Kompensatorelement an den Kompensator-Betriebsstrom IK angeschlossen und auf die stationäre Endspannung UK(ts) aufgeheizt. Es ist dann eine Konzentrationsmessung mit der vollen Meßgenauigkeit möglich.

Die Verarbeitungsfunktion läßt sich vorteilhafterweise als Exponentialfunktion der Form $UK(t) = UK(ts) \times (1-exp(-b(t,T) \times t))$ angeben. Versuche haben ergeben, daß sich der Spannungsverlauf am Kompensatorelement durch eine Funktion der oben genannten Art beschreiben läßt. Hierbei ist UK(t) die Momentanspannung zum Zeitpunkt t, UK(ts) die stationäre Endspannung nach der Aufheizzeit ts und b(t,T) ein Faktor, der abhängig ist von der Zeit t, der Gastemperatur T und gegebenenfalls von Umgebungseinflüssen wie relative Feuchte des Meßgases und Gasartzusammensetzung. Untersuchungen haben zu dem Ergebnis geführt, daß - mit Ausnahme der Zeit t und der Umgebungstemperatur T - andere Einflußparameter oberhalb einer Einschaltzeit der Zeitspanne t0 bis t1, keine signifikante Auswirkung auf den Kurvenverlauf der Verarbeitungsfunktion und damit auch auf b(t,T) haben.

Die Verarbeitungsfunktion wird zweckmäßigerweise in einem separat ablaufenden Kalibrierzyklus bestimmt. Hierzu wird das Kompensatorelement an den Kompensator-Betriebsstrom IK angeschlossen und die Spannungskurve UK(t) bei der Umgebungstemperatur T, beginnend vom Einschaltzeitpunkt t0, zu festgelegten Zeitpunkten aufgenommen und in einem Permanentspeicher der Meßeinrichtung abgelegt. Der stationäre Endwert UK(ts) ist dann der Spannungswert am Kompensatorelement nach der Einschaltzeit ts. Der stationäre Endwert liegt - je nach Kompensatortyp und konstruktiven Merkmalen des Sensorkopfes - etwa nach 30 Minuten vor.

Die Abhängigkeit der Verarbeitungsfunktion von der Umgebungstemperatur T wird ermittelt, indem der Kalibrierzyklus bei einer zweiten Umgebungstemperatur T1 wiederholt wird. Die Spannungskurve bei dieser Temperatur sei UK1(t). Da der Temperatureinfluß linear ist, können entsprechende Ausgleichsgeraden aus Stützwerten UK(te) und UK1(te) bei fester Zeit te errechnet werden. Mit diesen Ausgleichsgeraden können, jeweils für feste Zeiten te, Kompensator-Spannungswerte für Zwischentemperaturen bestimmt werden im Zeitintervall von t0 bis t1. Die Kalibrationskurven bei den Umgebungstemperaturen T und T1 können als für den Sensorkopf charakteristische Größen bei der Fertigung aufgenommen und in einen EPROM gespeichert werden. Später werden beide Komponenten in das zugehörige Meßgerät vor Ort eingesetzt. Weitere Kalibrierkurven können aufgenommen werden, wenn z.B. ein ensprechendes Startsignal von Hand bei Inbetriebnahme ausgelöst wird oder nach einem von der Meßeinrichtung vorgesehenen Zeitplan, oder bei einer Abweichung außerhalb der zulässigen Fehlerbandbereiche zwischen dem Meßwert der ersten Betriebsphase und dem Meßwert der zweiten Betriebsphase. Ist beispielsweise ein derartiger Kalibrierzyklus durch eine Plausibilitätsbetrachtung zwischen erster und zweiter Betriebsphase ausgelöst worden, wird bei der vorliegenden Gastemperatur T2 die Spannungskurve UK2(t) zu festgelegten Zeitpunkten aufgenommen und in dem Permanentspeicher der Meßeinrichtung abgelegt wird. Durch diesen Aufheizvorgang ist am Ende der Aufheizphase einerseits eine Bestimmung der Gaskonzentration mit voller Meßgenauigkeit möglich, andererseits liegt eine zusätzliche Kalibrierkurve für die Gastemperatur T2 vor.

In der zweiten Betriebsphase ist das Kompensatorelement vom Einschaltzeitpunkt t0 bis zum Zeitpunkt t1 während einer Teilaufheizphase an den Kompensator-Betriebsstrom IK angeschlossen. Das Zeitintervall t0 bis t1 beträgt 1 Sekunde bis 30 Sekunden - vorzugsweise 10 Sekunden -. Innerhalb dieses Zeitintervalls, zum Zeitpunkt te, oder am Ende des Zeitintervalls zum Zeitpunkt t1, wird mit der Verarbeitungsfunktion auf den stationären Endwert UK(ts) extrapoliert. Zur Berücksichtigung des Temperatureinflusses muß die Temperaturausgleichsgerade zum jeweiligen Extrapolationszeitpunkt te oder t1 ermittelt werden.

In der ersten Betriebsphase wird das Kompensatorelement mit dem Kompensator-Teilbetriebsstrom IK1 gespeist, der 20% bis 80% - vorzugsweise 50% - des Kompensator-Betriebsstroms IK beträgt. Bei einem Kompensator-Teilbetriebsstrom von 50% kann bereits nach wenigen Sekunden Aufheizzeit mit guter Genauigkeit auf den stationären Endwert UK (ts) extrapoliert werden. Dieser Wert stellt ein Optimum dar zwischen Leistungseinsparung in der ersten Betriebsphase und schneller Meßbereitschaft in der zweiten Betriebsphase.

Es ist zweckmäßig, bei erhöhten Anteilen brennbarer Gase in einem Gasgemisch, oder bei sich stark ändernden Konzentrationen, während einer dritten Betriebsphase das Kompensatorelement kontinuierlich mit dem Kompensator-Betriebsstrom IK zu speisen. Die dritte Betriebsphase kann nach Überschreiten eines zweiten Grenzwertes DU2 des zweiten Meßsignals U2 ausgelöst werden, der 40% bis 140% (UEG) - vorzugsweise 40% (UEG) - beträgt. Die dritte Betriebsphase kann auch nach einer festen, von der Meßeinrichtung vorgebbaren Zeit t3 ausgelöst werden.

Ein Ausführungsbeispiel der Erfindung wird anhand der Zeichnung dargestellt und im folgenden näher beschrieben.

Es zeigen:

Figur 1    ein Blockschaltbild der Meßeinrichtung zur Durchführung des Verfahrens,

Figur 2    den Verlauf der Spannung am Kompensatorelement im Taktbetrieb,

Figur 3    die Verarbeitungsfunktion zur Berechnung der Kompensator-Endspannung,

Figur 4    den Faktor b(t,T) in Abhängigkeit von der Temperatur T,

Figur 5    das Zeitverhalten der Spannungsdifferenz zwischen gemessener und extrapolierter Kompensator-Endspannung nach der Zeit ts von 30 Minuten.

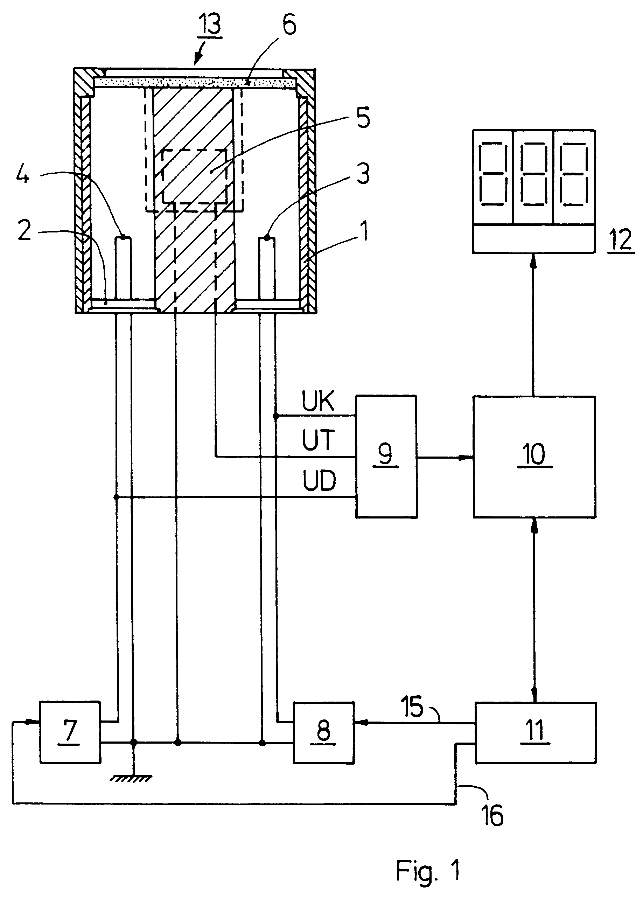

Die in Fig. 1 gezeigte Meßanordnung besteht aus einem Sensorkopf (13), einer Detektor-Spannungsversorgung (7), einer Kompensator-Spannungsversorgung (8), einem A/D-Wandler (9), einer Meßeinrichtung (10), einer Anzeigeeinheit (12) und einer Steuereinheit (11). Im Sensorkopf (13) sind auf einer Trägerplatte (2) ein Detektorelement (4) und ein Kompensatorelement (3) angeordnet. Das Meßgas dringt über eine Sintermetallscheibe (6) in den Sensorkopf (13) ein und wird am Detektorelement (4) katalytisch verbrannt. Die Meßgastemperatur innerhalb des Sensorkopfes (13) wird mit einem Platin-Widerstand als Temperaturfühler (5) gemessen. Die Meßsignale UD, UK und UT vom Detektorelement (4), Kompensatorelement (3) und Temperaturfühler (5) werden in dem A/D-Wandler (9) in digitale Signale umgesetzt und der Meßeinrichtung (10) zugeführt. Die Steuereinheit (11) ist sowohl mit der Meßeinrichtung (10) als auch mit den Spannungsversorgungen (7, 8) verbunden und gibt Strom-Steuerimpulse über Signalleitungen (15, 16) für das Detektorelement (4) und Kompensatorelement (3). Die Meßeinrichtung (10) beinhaltet im wesentlichen einen Mikropozessor und einen Permanentspeicher zur Abspeicherung von Verarbeitungsfunktionen, und eine Recheneinheit zur Berechnung von Spannungswerten für die Extrapolation der Kompensatorspannung und zur Umsetzung der gemessenen Spannungen am Kompensatorelement (3) und Detektorelement (4) in Gas-Konzentrationswerte.

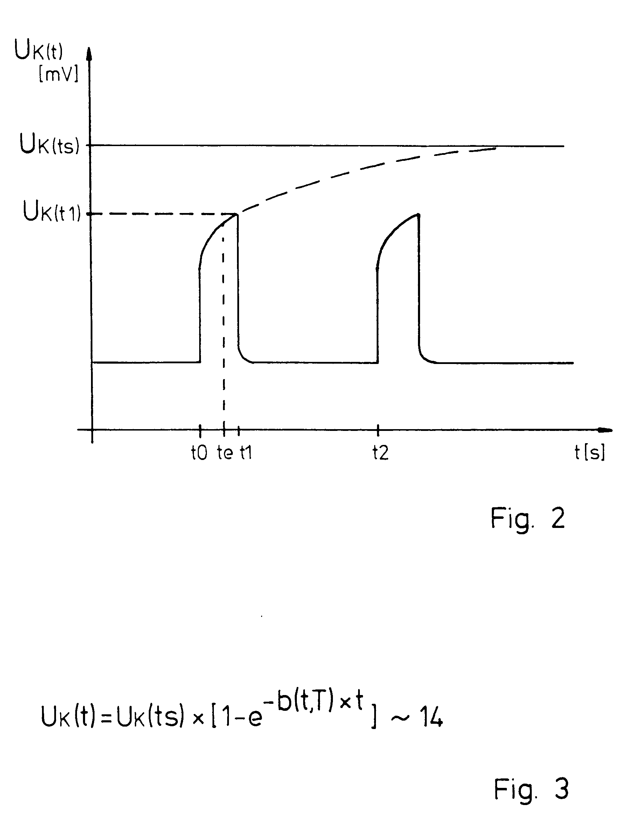

Fig. 2 zeigt den Spannungsverlauf UK(t) am Kompensatorelement (3) in Abhängigkeit von der Zeit t in einem Wechsel von der ersten Betriebsphase zur zweiten Betriebsphase. Zunächst wird das Kompensatorelement (3) in der ersten Betriebsphase mit einem Kompensator-Teilbetriebsstrom IK1 gespeist. Zum Zeitpunkt t0, dem Beginn der zweiten Betriebsphase, wird der Kompensator-Betriebsstrom IK angeschlossen. Die durchgezogene Linie in Fig. 2 gibt den Spannungsverlauf UK(t) am Kompensatorelement (3) wieder. Zum Zeitpunkt t1 ist die zweite Betriebsphase abgeschlossen, und es liegt wieder der Strom IK1 am Kompensatorelement an. Die gestrichelte Kurve oberhalb der Zeit t1 gibt den Spannungsverlauf UK(t) qualitativ an, wenn das Kompensatorelement (3) über den Zeitpunkt t1 hinaus auf den stationären Endwert UK(ts) aufgeheizt würde. Der stationäre Endwert ist hier nach etwa 30 Minuten erreicht. Die Zeit ts ist in dem gewählten Maßstab nicht darstellbar, da sie etwa 30 Minuten beträgt, während t1 nur 10 Sekunden ist. Zum Zeitpunkt t2 wird eine neue Aufheizphase eingeleitet. Im gezeigten Beispiel ist t1 etwa 10 Sekunden und t2 etwa 30 Sekunden bezogen auf den Einschaltzeitpunkt t0.

Fig. 3 gibt die Verarbeitungsfunktion (14) zur Extrapolation der Kompensatorspannung auf den stationären Endwert nach der Zeit ts an. Versuche haben ergeben, daß sich der Spannungsverlauf am Kompensatorelement (3) durch eine Exponentialfunktion der genannten Form beschreiben läßt. Hierbei ist UK(t) die Momentanspannung zum Zeitpunkt t, UK(ts) die stationäre Endspannung nach der Aufheizzeit ts und b(t,T) ein Faktor, der abhängig ist von der Zeit t, der Gastemperatur T und Umgebungseinflüssen wie relative Feuchte des Meßgases und Gasartzusammensetzung.

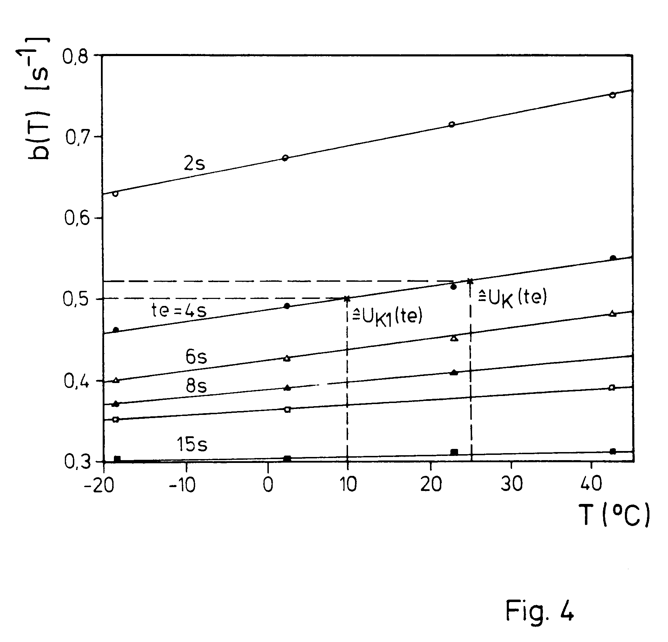

Fig. 4 gibt den Verlauf von b(t,T) als Funktion der Umgebungstemperatur T an. Als Kurvenparameter ist die Aufheizzeit t aufgetragen mit te als festem Zeitpunkt innerhalb der Aufheizzeit. Wegen des linearen Zusammenhangs von b(T) mit der Temperatur T, kann die Temperaturabhängigkeit mit einer Geraden approximiert werden, wenn mindestens zwei Stützwerte für eine entsprechende Aufheizzeit te vorliegen. Diese Stützwerte werden ermittelt, indem das Kompensatorelement in einem Kalibrierzyklus bei zwei unterschiedlichen Umgebungstemperaturen T und T1 an den Kompensator-Betriebsstrom IK angeschlossen wird und die Spannungen UK(t) aus dem ersten Kalibrierzyklus, und UK1(t) - aus dem zweiten Kalibrierzyklus, am Kompensatorelement (3) in festen Zeitabständen vom Startpunkt t0 bis zum stationären Endwert ts aufgenommen und in dem Permanentspeicher der Meßeinrichtung (10) abgelegt werden.

Die Stützwerte für die Temperaturausgleichsgerade sind dann die Spannungswerte von UK(te) und UK1(te) bei den Umgebungstemperaturen T und T1 zur gleichen Zeit te bezogen auf den jeweiligen Einschaltpunkt t0. Somit steht für jede Umgebungstemperatur T eine Extrapolationsfunktion vom Zeitpunkt te auf den stationären Endwert zum Zeitpunkt ts in der Meßeinrichtung (10) zur Verfügung. te ist ein vorgebbarer Zeitpunkt für die Extrapolation innerhalb des Intervalls von t0 bis t1. Die Extrapolation wird um so genauer, je größer die Zeit te gewählt wird, d. h. je näher te an t1 liegt.

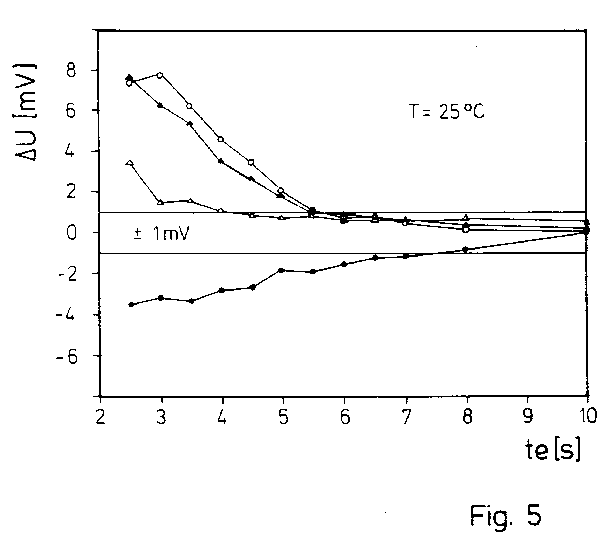

Die Güte der Extrapolation mit der Verarbeitungsfunktion (14) in Abhängigkeit von der Zeit te ist in Fig. 5 veranschaulicht. Berücksichtigt wurden hier auch unterschiedliche Umgebungsbedingungen wie relative Feuchte des Gases von z.B. 50% r.F. und 75% r.F. und Gaskonzentrationen $c(CH_4)$ von 2 Vol.% und $c(H_2)$ von 1Vol.%, bei konstanter Umgebungstemperatur T von 25 ° C. Für jede Umgebungsbedingung wurde die reale Aufheizkurve UK(t) gemessen und aus allen Kurven, bei jeweils gleichen Zeiten te, die b(te,T)-Werte bestimmt und für den Zeitpunkt te gemittelt. Mit den gemittelten b(te,T)-Werten wurde mit der Verarbeitungsfunktion (14) von unterschiedlichen Zeitpunkten te auf den stationären Endwert UK(ts) extrapoliert und der extrapolierte Wert mit dem gemessenem verglichen. Die Spannungsdifferenz Delta U ist auf der Ordinate aufgetragen. Der Bereich von ± 1 mV ist eine festgelegte Fehlergrenze für die Genauigkeit der Extrapolation. Aus Fig. 5 wird deutlich, daß die Umgebungseinflüsse nach einer gewissen Zeit abgeklungen sind und keinen nennenswerten Einfluß mehr auf die Extrapolation haben. Im gezeigten Beispiel liegt für Zeiten te größer 7 Sekunden der extrapolierte Endwert UK(ts) innerhalb der gewählten Fehlergrenze. Die Extrapolation ist zu jedem Zeitpunkt innerhalb des Zeitintervalls t0 bis t1 möglich, jedoch ist die Genauigkeit größer, je näher te an t1 liegt. Im folgenden wird ein typischer Meßablauf angegeben.

Zunächst wird ein Kalibrierzyklus ausgelöst, bei dem bei zwei unterschiedlichen Umgebungstemperaturen T und T1 das Kompensatorelement (3) an den Kompensator-Betriebsstrom IK angeschlossen wird. In festen Zeitabständen, z.B. vom Startpunkt t0 an alle 0,1 Sekunden, dann ab 20 Sekunden alle 5 Sekunden, werden die Spannungswerte UK(t) bei der Temperatur T und UK1(t) bei der Temperatur T1 am Kompensatorelement (3) gemessen und in einem Permanentspeicher der Meßeinrichtung (10) abgelegt. Spannungswerte bei Zwischentemperaturen werden von der Meßeinrichtung aus den beiden Kalibrierkurven durch lineare Extrapolation ermittelt, wobei aus beiden Kurven Stützwerte zur gleichen Zeit te verwendet werden. Die Kalibrierkurven können auch bei der Fertigung des Sensorkopfes (13) aufgenommen und in einem EPROM gespeichert werden, wobei dann beide Komponenten in das Meßgerät später eingesetzt werden.

Die Meßphase beginnt mit der ersten Betriebsphase - der Bereitschaftsphase - in der das Detektorelement (4) an den Detektor-Betriebsstrom ID angeschlossen ist, während das Kompensatorelement (3) mit dem Kompensator-Teilbetriebsstrom IK1 gespeist wird. So ist beispielsweise ID = 100 mA und IK1 50 mA. Das Meßsignal für die entspechende Gaskonzentration sei U1. Steigt die Konzentration eines brennbaren Gases in dem Meßgas - z.B. $CH_4$ in Luft -, erreicht das Meßsignal U1 einen ersten Grenzwert DU1, und es wird eine zweite Betriebsphase eingeleitet. Hier schaltet die Meßeinrichtung (10) für den Zeitraum t0 bis t1 den vollen Kompensator-Betriebsstrom IK an das Kompensatorelement und heizt dieses auf. Gleichzeitig wird mit dem Temperaturfühler (5) die Umgebungstemperatur T im Sensorkopf (13) gemessen. Zum Zeitpunkt te wird mit den im Permanentspeicher der Meßeinrichtung (10) gespeicherten Kalibrierkurven auf den stationären Endwert der Kompensatorspannung UK(ts) extrapoliert. Bereits zum Zeitpunkt te kann also so gemessen werden, als sei das Kompensatorelement (3) auf den stationären Endwert aufgeheizt worden. Ist die zweite Betriebsphase durch Überschreiten des ersten Grenzwertes DU1 ausgelöst worden und die Gaskonzentration im Sensorkopf während der zweiten Betriebsphase unverändert geblieben, muß durch die Meßeinrichtung (10) der weitere Ablauf so beeinflußt werden, daß die zweite Betriebsphase nicht anschließend wieder ausgelöst wird. Dieser Ablauf kann beispielsweise so erfolgen, daß der erste Grenzwert DU1 für eine befristete Zeit angehoben wird, oder erst nach einem von der Meßeinrichtung (10) vorgebbaren Intervall wieder ausgelöst werden kann. Der Wechsel zwischen der ersten und zweiten Betriebsphase kann auch zu festen, von der Meßeinrichtung (10) vorgebbaren Zeiten erfolgen, indem im Wechsel, nach z.B. 20 Sekunden Bereitschaftsphase eine Meßphase von 10 Sekunden folgt. Der Vorteil besteht darin, daß bei kontinuierlicher Meßbereitschaft von 30 Sekunden nur während 10 Sekunden die volle Leistung benötigt wird.

Bei erhöhten Konzentrationen von brennbaren Gasen oder bei schnell wechselnden Gaskonzentrationen wird dann in die dritte Betriebsphase umgeschaltet, in der das Detektorelement (4) und das Kompensatorelement (3) ständig mit den Betriebsströmen ID und IK gespeist werden. Diese Betriebsphase kann auch von der Meßeinrichtung (10) zu festgelegten Zeitpunkten, z.B. jede Stunde, eingeschaltet werden, um Referenzmeßwerte mit voller Meßgenauigkeit zu gewinnen, die auch Rückschlüsse auf mögliche Fehlerquellen im Meßsystem gestatten. Eine Fehlererkennung ist auch beispielsweise in dem Wechselspiel von erster und zweiter Betriebsphase möglich, indem die Einzelmeßsignale U1 der zweiten Betriebsphase miteinander korreliert und auf Plausibilität geprüft werden. Außerdem besteht die Möglichkeit, innerhalb der zweiten Betriebsphase mehrere Extrapolationen zu unterschiedlichen Zeitpunkten te durchzuführen und diese extrapolierten Werte miteinander zu korrelieren. Auf diese Weise können stochastische Schwankungen verringert werden.

**Patentansprüche**

1. Verfahren für den Betrieb einer Meßanordnung zum Nachweis von brennbaren Gasen in einem Gasgemisch mit einer Anzeigeneinheit, einer Meßeinrichtung, einem Detektorelement und einem ansteuerbaren Kompensatorelement, dadurch gekennzeichnet, daß während einer ersten Betriebsphase das Detektorelement (4) mit dem Detektor-Betriebsstrom ID gespeist wird, während das Kompensatorelement (3) mit einem Kompensator-Teilbetriebsstrom IK1 betrieben wird und aus den Spannungen am Detektorelement (4) UD und Kompensatorelement (3) UK ein erstes Meßsignal U1 zur Gasanalyse gebildet wird und daß in einer zweiten Betriebsphase das Kompensatorelement (3) vom Einschaltzeitpunkt t0 bis zum Zeitpunkt t1 in einer Teil-Aufheizphase an den Kompensator-Betriebsstrom IK angeschlossen ist und der stationäre Endwert der Kompensatorspannung UK(ts) am Ende der Aufheizphase aus dem Anstieg der Kompensatorspannung UK(t) im Zeitintervall t0 bis t1 mit einer Verarbeitungsfunktion (14) extrapoliert wird und mit der extrapolierten Endspannung UK(ts) und der Spannung UD am Detektorelement (4) ein zweites Meßsignal U2 zur Gasanalyse gebildet wird.

2. Verfahren nach Anspruch 1, dadurch gekennzeichnet, daß zur Erfassung der Temperatur T am Detektorelement (4) und Kompensatorelement (3) ein Temperaturfühler (5) im Sensorkopf (13) vorgesehen ist.

3. Verfahren nach Anspruch 1 oder 2, dadurch gekennzeichnet, daß die zweite Betriebsphase nach Überschreiten eines ersten Grenzwertes DU1 des ersten Meßsignals U1 ausgelöst wird.

4. Verfahren nach Anspruch 3, dadurch gekennzeichnet, daß der erste Grenzwert DU1 im Bereich 1% bis 20% - vorzugsweise 5% - der unteren Explosionsgrenze (UEG) liegt.

5. Verfahren nach Anspruch 1, dadurch gekennzeichnet, daß die zweite Betriebsphase nach einer von der Meßeinrichtung (10) vorgebbaren Zeit t2 ausgelöst wird.

6. Verfahren nach einem der Ansprüche 1 bis 5, dadurch gekennzeichnet, daß die Verarbeitungsfunktion (14) eine Exponentialfunktion der Form UK(t) = UK(ts) x (1-exp(-b(t,T) x t)) ist, wobei b(t,T) ein temperatur- und zeitabhängiger Faktor ist, der von Umgebungseinflüssen abhängt.

7. Verfahren nach einem der Ansprüche 1 bis 6, dadurch gekennzeichnet, daß die Verarbeitungsfunktion (14) in einem separat ablaufenden Kalibrierzyklus bestimmt wird, in dem das Kompensatorelement (3) mit dem Kompensator-Betriebsstrom IK auf die stationäre Endtemperatur aufgeheizt und in vorwählbaren Zeitabschnitten die Spannungskurve UK(t) bei konstanter Temperatur T aufgenommen und in einem Permanentspeicher der Meßeinrichtung (10) abgelegt wird.

8. Verfahren nach Anspruch 7, dadurch gekennzeichnet, daß zur Ermittlung des Temperatureinflusses von b(t, T) eine zweite Spannungskurve UK1(t) bei einer zweiten Umgebungstemperatur T1 aufgenommen wird und die jeweilige Temperatur-Ausgleichsgerade aus Stützwerten UK(te) und UK1(te) bei fester Zeit te errechnet wird.

9. Verfahren nach Anspruch 7 oder 8, dadurch gekennzeichnet, daß der Kalibrierzyklus von der Meßeinrichtung (10) selbsttätig auslösbar ist.

10. Verfahren nach einem der Ansprüche 1 bis 8, dadurch gekennzeichnet, daß das Zeitintervall t0 bis t1 in der zweiten Betriebsphase 1 Sekunde bis 30 Sekunden - vorzugsweise 10 Sekunden - beträgt.

11. Verfahren nach einem der Ansprüche 1 bis 10, dadurch gekennzeichnet, daß der Kompensator-Teilbetriebsstrom IK1 20% bis 80% - vorzugsweise 50% - des Kompensator-Betriebsstroms IK beträgt.

12. Verfahren nach einem der Ansprüche 1 bis 11, dadurch gekennzeichnet, daß während einer dritten Betriebsphase das Kompensatorelement (3) kontinuierlich mit dem Kompensator-Betriebsstrom IK gespeist wird.

13. Verfahren nach Anspruch 12, dadurch gekennzeichnet, daß die dritte Betriebsphase beim Überschreiten eines zweiten Grenzwertes DU2 des zweiten Meßsignals U2 ausgelöst wird.

14. Verfahren nach Anspruch 13, dadurch gekennzeichnet, daß der zweite Grenzwert DU2 40% bis 140% (UEG) - vorzugsweise 40% (UEG) - beträgt.

15. Verfahren nach Anspruch 12, dadurch gekennzeichnet, daß die dritte Betriebsphase nach einer von der Meßeinrichtung (10) vorgebbaren Zeit t3 ausgelöst wird.

**Claims**

1. Method for operation a measuring arrangement for the detection of combustible gases in a gas mixture, having an indicator unit, a measuring device, a detector element and a controllable compensator element, characterised in that during a first phase of operation, the detector operating current ID is fed to the detector element (4), while the compensator element (3) is operated with a compensator partial-operating current IK1, and a first measuring signal U1 for the gas analysis is formed from the voltages at the detector element (4) UD and compensator element (3) UK, and in that in a second phase of operation, the compensator element (3) is connected to the compensator operating current IK from the instant of switching on t0 to the instant t1 in a partial heating phase, and the constant final value of the compensator voltage UK(ts) at the end of the heating phase is extrapolated from the rise in the compensator voltage UK(t) during the time interval t0 to t1 using a processing function (14), and a second measuring signal U2 for the gas analysis is formed with the extrapolated final voltage UK(ts) and the voltage UD at the detector element (4).

2. Method according to claim 1, characterised in that in order to detect the temperature T at the detector element (4) and compensator element (3), a temperature probe (5) is provided in the sensor head (13).

3. Method according to claim 1 or 2, characterised in that the second phase of operation is triggered after a first limiting value DU1 of the first measuring signal U1 has been exceeded.

4. Method according to claim 3, characterised in that the first limiting value DU1 is in the region of 1% to 20% - preferably 5% - of the lower explosion limit (LEL).

5. Method according to claim 1, characterised in that the second phase of operation is triggered after a time t2, which can be pre-determined by the measuring device (10).

6. Method according to one of the claims 1 to 5, characterised in that the processing function (14) is an exponential function of the form

$$UK(t) = UK(ts) \times (1-\exp(-b(t,T) \times t)),$$

wherein $b(t,T)$ is a temperature-dependent and time-dependent factor which is dependent on ambient influences.

7. Method according to one of the claims 1 to 6, characterised in that the processing function (14) is determined in a calibrating cycle, which runs separately and in which the compensator element (3) is heated up with the compensator operating current IK to the constant final temperature, and, in pre-selectable time periods, the voltage curve UK(t) at constant temperature T is recorded and deposited in a permanent memory of the measuring device (10).

8. Method according to claim 7, characterised in that in order to establish the influence of temperature of b(t,T), a second voltage curve UK1-(t) at a second ambient temperature T1 is recorded and the respective temperature-equalizing straight line is calculated from supporting values UK(te) and UK1(te) at fixed time te.

9. Method according to claim 7 or 8, characterised in that the calibrating cycle of the measuring device (10) can be automatically triggered.

10. Method according to one of the claims 1 to 8, characterised in that the time interval t0 to t1 in the second phase of operation amounts to 1 second to 30 seconds, preferably 10 seconds.

11. Method according to one of the claims 1 to 10, characterised in that the compensator partial-operating current IK1 amounts to 20% to 80% - preferably 50% - of the compensator operating current IK.

12. Method according to one of the claims 1 to 11, characterised in that during a third phase of operation, the compensator operating current IK is fed continuously to the compensator element (3).

13. Method according to claim 12, characterised in that the third phase of operation is triggered when a second limiting value DU2 of the second measuring signal U2 is exceeded.

14. Method according to claim 13, characterised in that the second limiting value DU2 amounts to 40% to 140% (LEL), preferably 40% (LEL).

15. Method according to claim 12, characterised in that the third phase of operation is triggered after a time t3, which can be pre-determined by the measuring device (10).

**Revendications**

1. Procédé permettant de faire fonctionner un système de mesure servant à détecter des gaz combustibles dans un mélange gazeux, comprenant une unité d'affichage, un dispositif de mesure, un détecteur et un compensateur actionnable, caractérisé en ce que lors d'une première phase de fonctionnement, le détecteur (4) est alimenté avec le courant de service du détecteur ID, tandis que le compensateur (3) fonctionne avec un courant de service partiel du compensateur IK1 et qu'un premier signal de mesure U1 destiné à l'analyse du gaz est formé à partir des tensions au niveau du détecteur (4) UD et du compensateur (3) UK, et en ce que, lors d'une deuxième phase de fonctionnement, le compensateur (3), entre l'instant de mise en route to et l'instant T1, est raccordé, au cours d'une phase partielle de chauffage, au courant de service du compensateur IK, que la valeur finale fixe de la tension du compensateur UK(ts), à l'issue de la phase de chauffage, est extrapolée à partir de l'élévation de la tension du compensateur UK(t), dans l'intervalle entre l'instant t0 et t1, avec une fonction de traitement (14), et qu'un deuxième signal de mesure U2 pour l'analyse du gaz est formé avec la tension finale extrapolée UK(ts) et la tension régnant au niveau du détecteur (4) UD.

2. Procédé selon la revendication 1, caractérisé en ce qu'une sonde de température (5) est prévue dans la tête du capteur (13) pour la détection de la température T au niveau du détecteur (4) et du compensateur (3).

3. Procédé selon la revendication 1 ou 2, caractérisé en ce que la deuxième phase de fonctionnement est déclenchée une fois qu'est dépassée une première valeur seuil DU1 du premier signal de mesure U1.

4. Procédé selon la revendication 3, caractérisé en ce que la première valeur seuil DU1 se situe entre 1% et 20% - de préférence 5% - de la limite inférieure d'explosion (LIE).

5. Procédé selon la revendication 1, caractérisé en ce que la deuxième phase de fonctionnement est déclenchée après un temps t2 susceptible d'être déterminé par le dispositif de mesure (10).

6. Procédé selon l'une des revendications 1 à 5, caractérisé en ce que la fonction de traitement (14) est une fonction exponentielle de la forme UK(t) = UK (ts) x (1-exp(-b(t, T) x t)), sachant que b(t, T) est un facteur dépendant de la température et du temps, qui est soumis à l'influence du milieu environnant.

7. Procédé selon l'une des revendications 1 à 6, caractérisé en ce que la fonction de traitement (14) est déterminée au cours d'un cycle de calibrage séparé, dans lequel le compensateur (3), à l'aide du courant de service du compensateur IK, est chauffé à la température finale fixe et la courbe de tension UK(t), à des moments susceptibles d'être présélectionnés, est relevée à température constante T et est entrée dans une mémoire permanente du dispositif de mesure (10).

8. Procédé selon la revendication 7, caractérisé en ce que, pour déterminer l'influence de la température de b(t, T), une deuxième courbe de tension UK1(t) est enregistrée à une deuxième température ambiante T1 et la droite de compensation correspondante de la température est calculée à partir de valeurs auxiliaires UK(te) et UK1(te), avec un temps fixe.

9. Procédé selon la revendication 7 ou 8, caractérisé en ce que le cycle de calibrage est susceptible d'être actionné automatiquement par le dispositif de mesure (10).

10. Procédé selon l'une des revendications 1 à 8, caractérisé en ce que l'intervalle de temps entre t0 et t1, au cours de la deuxième phase de fonctionnement, est de 1 à 30 secondes - de préférence 10 secondes.

11. Procédé selon l'une des revendications 1 à 10, caractérisé en ce que le courant de service partiel du compensateur IK1 représente 20% à 80% - de préférence 50% - du courant de service du compensateur IK.

12. Procédé selon l'une des revendications 1 à 11, caractérisé en ce qu'au cours d'une troisième phase de fonctionnement, le compensateur (3) est alimenté de façon continue avec le courant de service du compensateur IK.

13. Procédé selon la revendication 12, caractérisé en ce que la troisième phase de fonctionnement est déclenchée lorsqu'est dépassée une deuxième valeur seuil DU2 du deuxième signal de mesure U2.

14. Procédé selon la revendication 13, caractérisé en ce que la deuxième valeur seuil DU2 représente 40% à 140% (LIE) - de préférence 40% (LIE).

15. Procédé selon la revendication 12, caractérisé en ce que la troisième phase de fonctionnement est déclenchée après que se soit écoulé un temps t3, susceptible d'être prédéterminé par le dispositif de mesure (10).

UK

UT

UD

Fig. 1

Fig. 2

$$U_K(t) = U_K(ts) \times [1 - e^{-b(t,T) \times t}] \sim 14$$

Fig. 3

Fig. 4

Fig. 5